# FASCICULE DE BREVET EUROPEEN

(11) **EP 2 545 158 B1**
(45) Date de publication et mention de la délivrance du brevet: **30.07.2014**
(21) Numéro de dépôt: 11715934.3
(22) Date de dépôt: 11.03.2011
(51) Int. Cl.: C12N 1/14, A01N 63/04, C12R 1/645

(54) **TRAITEMENT DES VÉGÉTAUX CONTRE L'INFECTION PAR DES OOMYCÈTES**
BEHANDLUNG VON PFLANZEN GEGEN EIPILZ-INFEKTIONEN
TREATMENT OF PLANTS AGAINST OOMYCETE INFECTION

(30) Priorité: 11.03.2010 FR 1051767
(43) Date de publication de la demande: 16.01.2013
(73) Titulaire: Institut National de la Recherche Agronomique, 75338 Paris Cedex 07 (FR)
(72) Inventeur: GALIANA, Eric, F-06600 Antibes (FR); PONCHET, Michel, F-06600 Antibes (FR); MARAIS, Antoine, F-06600 Antibes (FR)
(74) Mandataire: Chauveau, Ariane
(86) Numéro de dépôt international: PCT/FR2011/000128
(87) Numéro de publication internationale: WO 2011/110758

(56) Documents cités:
- WO-A1-95/31106
- WO-A2-2010/009241
- US-B1- 6 544 512
- GALIANA ERIC ET AL: "Phytophthora parasitica biofilm formation: installation and organization of microcolonies on the surface of a host plant.", ENVIRONMENTAL MICROBIOLOGY AUG 2008 LNKD- PUBMED:18430009, vol. 10, no. 8, août 2008 (2008-08), pages 2164-2171, XP002605049, ISSN: 1462-2920
- KAEWCHAI S ET AL: "Mycofungicides and fungal biofertilizers", FUNGAL DIVERSITY, vol. 38, septembre 2009 (2009-09), pages 25-50, XP002605050, ISSN: 1560-2745
- DATABASE EMBL [Online] 5 juillet 2010 (2010-07-05), "Phoma sp. Y3 EG-2010 18S ribosomal RNA gene, partial sequence.", XP002648120, extrait de EBI accession no. EM_FUN:HM161743 Database accession no. HM161743

## Description

L'invention concerne un micro-organisme utile pour le traitement des végétaux contre l'infection causée par des oomycètes phytopathogènes. L'invention a pour but d'utiliser d'autres sources de traitements que ceux habituellement utilisés tels que des traitements chimique ou génétique.

Les oomycètes représentent un phylum de protistes filamenteux comprenant environ 500 espèces. Ce sont des organismes aquatiques non photosynthétiques qui, bien que ressemblant aux champignons, en sont éloignés. Des études moléculaires récentes ont permis de mieux les classer dans le taxon des Straménopiles. Ils sont caractérisés par l'existence, au cours de leur cycle, d'une cellule biflagellée.

Les oomycètes vivent dans l'eau, sur des déchets organiques et des cadavres des petits animaux. Certaines espèces vivent dans le sol en saprophytes sur les débris organiques. Plusieurs espèces sont des pathogènes majeurs de plantes. Parmi les oomycètes phytopathogènes, on connait le genre *Pythium* qui comprend de nombreuses espèces parasites de plantes et quelques autres parasites d'animaux. Le genre *Phytophthora* est également responsable de maladies chez les végétaux sauvages et cultivés. *Plasmopara viticola* est l'agent du mildiou de la vigne.

Par exemple, l'espèce *Phytophthora parasitica (P. parasitica)* engendre une maladie qui touche les végétaux, en particulier les plantes cultivées telles que la tomate, le piment, l'aubergine, les agrumes, le cacao, le tabac. Plus précisément, l'espèce *P. parasitica* est responsable chez le tabac de l'apparition du syndrome du « black shank ».

Les maladies végétales provoquées par l'infection par des oomycètes se manifestent par des symptômes variés, foliaires ou racinaires (pourriture pied noir, nanisme, taches brunes, puis flétrissement général de la feuille, d'un rameau ou de toute la plante). Les oomycètes se trouvent fréquemment associés à la rhizosphere et peuvent être transmis par le sol.

Ainsi, parmi les oomycètes, beaucoup d'entre eux sont donc des micro-organismes phytopathogènes, constituant une contrainte majeure pour l'agriculture mondiale et l'environnement causant des pertes mondiales considérables (de 10 à 60% selon les cultures) en particulier dues à *Phytophthora parasitica, sojae* et *ramorum, Plasmopara halstedii* et *viticola.*

Pour limiter l'impact des maladies provoquées par les oomycètes des fongicides sont en général utilisés, en particulier le metalaxyl, mais aussi des traitements à base de cuivre, des fongicides de contact (manèbe, mancozèbe, fluazinam, ...), des fongicides pénétrants (cymoxanil), des fongicides diffusants (dimétomorph, propamocarbe) ou des fongicides systémiques (oxadyxil). Le nombre de substances actives disponibles est toutefois réduit ; de plus, non seulement des risques de résistances d'oomycètes apparaissent mais ces produits peuvent potentiellement être nuisibles pour l'environnement.

Une autre alternative consiste en la création de variétés végétales résistantes aux maladies, sur la base d'un criblage des ressources génétiques disponibles selon des méthodes classiques ou par voie biotechnologique, comme exposé par exemple dans le brevet US2008 022423.

Mais, de la même manière que pour les produits chimiques, les oomycètes ont la capacité de contourner les résistances variétales ; de plus, le temps de développement d'une variété résistante issue de ces programmes d'amélioration génétique s'avère assez long.

Des traitements faisant intervenir des micro-organismes comme agents de lutte biologique contre divers champignons phytopathogènes sont également connus; par exemple, les brevets WO95/20879, WO95/31106, WO03/065811, KR2003/0075092, WO2010/009241 enseignent respectivement l'utilisation de métabolites produits par *Trichoderma,* d'une souche de *Fusarium,* de souches de *Bacillius cereus* et *subtilis,* d'une souche de *Paenibacillus,* d'une combinaison *de Trichoderma* et *Bacillus amyloliquefasciens,* afin de lutter notamment contre *Phytophthora.*

Toutefois les professionnels disposent d'encore peu de méthodes de contrôle biologique des oomycetes malgré le regain d'intérêt pour ces méthodes alternatives. C'est dans ce contexte que les inventeurs ont cherché une solution alternative aux traitements chimiques sans les inconvénients des autres méthodes citées ci-dessus.

Par un procédé tout à fait original d'identification des micro-organismes capables de cohabiter au sein d'un biofilm avec au moins une espèce donnée d'oomycète phytopathogène, les inventeurs ont isolé une souche de micro-organisme particulière qui a pour effet d'empêcher le développement d'une infection par ladite espèce d'oomycète phytopathogène. Plus précisément, les inventeurs ont découvert que cette souche de micro-organisme permettait de contrôler la croissance dudit oomycète phytopathogène. Le contrôle de la croissance s'effectue en inhibant au moins partiellement la croissance de l'oomycète phytopathogène. Les inventeurs se sont en effet intéressés à la flore microbienne présente dans la rhizosphère d'une plante. A la surface d'une plante hôte, l'oomycète forme un biofilm auquel s'associent d'autres micro-organismes provenant notamment de la rhizosphère. Par biofilm on entend une pellicule formée par un regroupement d'oomycètes, lesdits oomycètes générant une matrice protectrice adhésive composée de substances polymères et de substances dites chémo-attractives qui vont attirer les divers micro-organismes provenant de la rhizosphère.

Les micro-organismes que l'on retrouve associés dans les biofilms ont été sélectionnés au cours du temps pour leur capacité à cohabiter avec l'oomycète phytopathogène. Ainsi ont probablement été sélectionnés des micro-organismes capables de se développer en utilisant comme seule ressource nutritive celle apportée par l'oomycète phytopathogène ; parmi ces mêmes micro-organismes certains sont éventuellement capables d'inhiber au moins partiellement la croissance de l'oomycète auquel ils sont associés.

En mélangeant les micro-organismes de la rhizosphère avec l'oomycète phytopathogène, les inventeurs ont isolé et caractérisé une nouvelle souche de micro-organisme qui inhibe le développement de l'oomycète.

Dans un exemple de réalisation de l'invention, l'oomycète phytopathogène est *Phytophthora parasitica* (*P. parasitica*). Mais il pourrait s'agir d'une toute autre espèce d'oomycète phytopathogène.

L'invention a donc pour objet une souche de champignon filamenteux du genre *Phoma* déposée à la Collection Nationale de Cultures de Microorganismes de l'Institut Pasteur le 25 février 2010 sous le n°CNCM I-4278.

Une analyse moléculaire a permis d'établir que cette souche comporte un ARN ribosomal 18S ou ARNr 18S codé par un gène de séquence nucléotidique SEQ ID N°1. Cette séquence nucléotidique SEQ ID N°1 présente 98% d'identité de séquence avec la séquence nucléotidique codant pour l'ARNr 18S de *Phoma herbarum.*

La souche de *Phoma* précédemment identifiée présente en outre les caractéristiques suivantes :
- elle inhibe au moins partiellement la croissance des oomycètes phytopathogènes,
- elle inhibe la croissance des oomycètes du genre Phytophthora,
- elle inhibe la croissance de Phytophthora parasitica.

Dans le même exemple de réalisation de l'invention, les inventeurs ont découvert qu'un surnageant de culture de la souche de *Phoma* objet de l'invention inhibe la croissance de *P. parasitica.* Mais il est entendu que ce surnageant pourrait également inhiber au moins partiellement la croissance d'autres espèces d'oomycètes.

L'invention a donc également pour objet un surnageant de culture de la souche précédemment décrite, ledit surnageant de culture inhibant au moins partiellement la croissance des oomycètes phytopathogènes.

L'invention présente en outre les caractéristiques suivantes :
- le surnageant comporte au moins un métabolite de taille inférieure ou égale à une valeur comprise entre 0,18µm et 0,22µm, de préférence inférieure ou égale à 0,2µm.
- le surnageant de culture inhibe la croissance de *Phytophthora parasitica.*

L'invention a également pour objet un procédé d'obtention du surnageant précédemment mentionné, ledit procédé comprenant les étapes suivantes
- mise en culture de la souche précédemment décrite,
- mise en suspension de la culture obtenue, caractérisé en ce qu'il comporte l'étape suivante
- filtration de la suspension au travers d'un tamis de 0,18µm à 0,22µm, de préférence 0,2µm,
- récupérer la solution filtrée.

En variante, préalablement à la filtration de la suspension, le procédé d'obtention prévoit de centrifuger la suspension entre 1500g et 6000g pendant 1 à 10 minutes, de préférence à 2000g pendant 2 minutes.

L'invention a pour objet également un autre procédé d'obtention du surnageant précédemment mentionné, comprenant les étapes suivantes
- mise en culture de la souche précédemment décrite,
- mise en suspension de la culture obtenue, caractérisé en ce qu'il comporte l'étape suivante
- centrifuger la suspension entre 1500g et 6000g pendant 1 à 10 minutes, de préférence à 2000g pendant 2 minutes, puis
- récupérer le surnageant.

La souche précédemment décrite peut être utilisée pour la fabrication d'une composition phytosanitaire destinée à traiter des végétaux contre l'infection par des oomycètes. Par composition phytosanitaire, on entend une composition comprenant outre la souche de *Phoma* et/ou un surnageant de culture, comme substance active, des additifs acceptables en agriculture.

Dans le même exemple de l'invention non limitatif; l'une des espèces d'oomycètes contre laquelle est active la souche est l'espèce *P. parasitica.* Mais il pourrait s'agir d'une autre espèce de *Phytophthora* ou d'une autre espèce d'oomycète.

L'invention a donc aussi pour objet une composition phytosanitaire, caractérisée en ce qu'elle comprend la souche de *Phoma* précédemment décrite.

Le surnageant de culture de la souche de *Phoma* déposée et identifiée ci-dessus peut également être utilisé pour la fabrication d'une composition phytosanitaire destinée à traiter des végétaux contre l'infection par des oomycètes phytopathogènes.

L'invention a donc aussi pour objet une composition phytosanitaire, caractérisée en ce qu'elle comprend un surnageant de culture comme précédemment décrit.

Notamment, les végétaux susceptibles d'être traités peuvent être une plante de tabac, tomate, pomme de terre, poivron, vigne, tournesol, des arbres fruitiers, ou tout autre type de végétal susceptible d'être infecté par des espèces oomycètes phytopathogènes.

L'invention a aussi pour objet une composition phytosanitaire, caractérisée en ce qu'elle comprend la souche de *Phoma* précédemment décrite.

L'invention a aussi pour objet une composition phytosanitaire, caractérisée en ce qu'elle comprend un surnageant de culture comme précédemment décrit.

L'invention a aussi pour objet une composition phytosanitaire, caractérisée en ce qu'elle comprend la souche de *Phoma* précédemment décrite et le surnageant de culture comme précédemment décrit.

L'invention a aussi pour objet l'utilisation de la souche de *Phoma* précédemment décrite pour la fabrication d'une composition phytosanitaire destinée à traiter des végétaux contre l'infection par des oomycètes phytopathogènes.

L'invention a aussi pour objet l'utilisation du surnageant de culture précédemment décrit pour la fabrication d'une composition phytosanitaire destinée à traiter des végétaux contre l'infection par des oomycètes phytopathogènes.

L'invention a enfin pour objet l'utilisation de la souche de *Phoma* précédemment décrite et du surnageant de culture précédemment décrit pour la fabrication d'une composition phytosanitaire destinée à traiter des végétaux contre l'infection par des oomycètes phytopathogènes.

L'invention sera mieux comprise à la lecture de la description qui suit et à l'examen des figures qui l'accompagnent. Celles-ci ne sont présentées qu'à titre indicatif et nullement limitatif de l'invention. Les figures montrent :
Figure 1 : Une représentation schématique d'une formation d'une communauté de micro-organismes prélevés à partir d'une rhizosphère d'une plante et d'une micro-colonie de *P. parasitica ;*
Figure 2: Une représentation schématique d'une formation d'un biofilm à partir d'une colonie de *P. parasitica ;*
Figure 3 : Une représentation schématique d'un procédé de sélection de micro-organismes capables de vivre en présence de *P. parasitica ;*
Figure 4 : Des représentations schématiques d'une co-infection *in vitro* et *in planta* de *P. parasitica* et de micro-organismes capables de vivre en présence de *P. parasitica ;*
Figure 5A : Une photographie au microscope optique d'un milieu de culture comprenant seulement un filtrat comprenant de l'eau et l'espèce *P*. *parasitica ;*
Figure 5B : Une photographie au microscope optique d'un milieu de culture comprenant *P. parasitica* et un surnageant de culture de micro-organismes provenant de l'isolat I3 sélectionné pour la capacité des mêmes micro-organismes provenant de l'isolat I3 à survivre en présence de *P*. *parasitica* ;
Figure 6 : Une photographie d'une feuille de tabac qui été mise au contact de zoospores de *P. parasitica* seuls (Pp) ou au contact de zoospores de *P. parasitica* mélangés avec des spores provenant de différents micro-organismes (I1 : Penicillium, I2 : *Aspergillus* et I3 : souche de *Phoma*), et
Figure 7 : Une représentation graphique en pourcentage de l'effet d'un surnageant de culture de micro-organismes sur la germination de zoospores de *P. parasitica* (I3) et de l'effet d'un autre filtrat contenant de l'eau sur la croissance de *Phytophthora parasitica* (C), lesdits micro-organismes provenant d'un isolat I3 sélectionné pour la capacité des micro-organismes contenus dans cet isolat à survivre en présence de *P. parasitica.*

### 1 - Matériels et méthodes

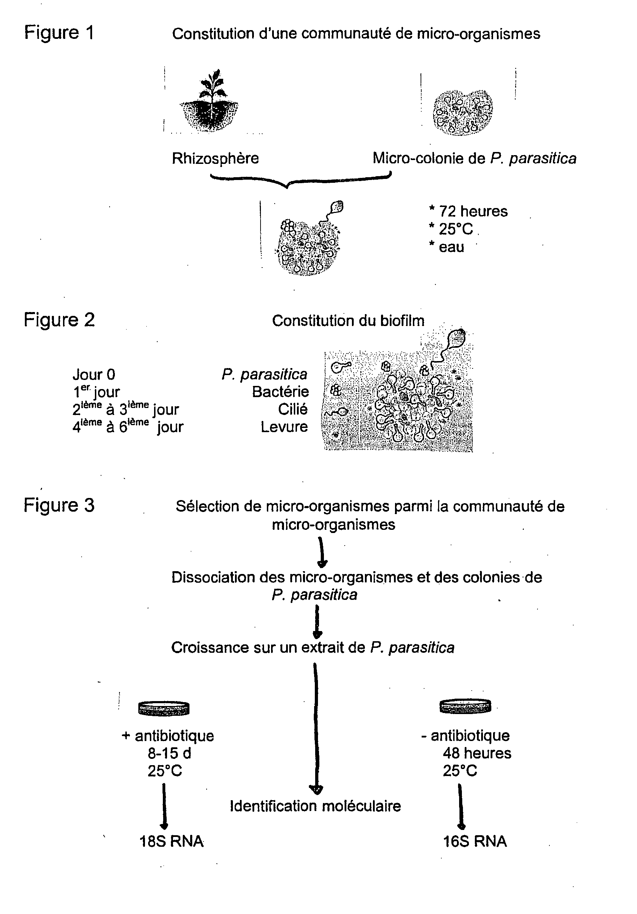

### 1.1. Constitution de la communauté (Figure 1)

Une plante de tabac âgée de 5 semaines a été mise en culture dans un terreau vendu dans le commerce et cultivée à 24°C dans un phytotron, avec une photopériode de 16 heures et une intensité lumineuse de 100µEm²sec⁻¹. Après 5 semaines de croissance, des échantillons de sols ont été prélevés à partir de la rhizosphère de cette même plante. Ces échantillons ont été mixés avec de l'eau stérile (1/5, WN). La flore microbienne rhizosphérique (taille des microorganismes < 100 µm) a été obtenue par deux filtrations successives au travers d'un tamis de 100µm de diamètre. Après une période rapide de décantation, le surnageant (5ml) obtenu à partir du filtrat a été incubé à 24°C avec des micro-colonies de *Phytophthora parasitica* (ou *P. parasitica*) préparées comme décrit dans E. Galiana, S. Fourré, G. Engler, Environ. Microbiol. 10, 2164-2171 (2008) et lavées trois fois à l'eau. La cinétique de colonisation des micro-colonies de *P. parasitica* par des micro-organismes rhizosphériques a été déterminée après observation au microscope optique.

### 1.2. Sélection de la communauté (figures 2 et 3)

Le mélange de micro-organismes rhizosphèriques et de microcolonies de *P. parasitica* forme un biofilm. Après trois jours d'incubation, les biofilms obtenus sont rincés trois fois dans de l'eau. Les micro-organismes et les micro-colonies de *P. parasitica* formant les biofilms sont ensuite dissociés les uns des autres avec douceur au travers de l'orifice d'une pipette Pasteur. La suspension de cellules de micro-organismes en résultant est incubée sur un gel d'agar d'une boîte de Petri. Le gel d'agar contient un extrait de *P*. *parasitica* comme seul source de nutriment (extrait brut de *P. parasitica* 10g/L ; NaCl 10g/L ; agar 1.5% (P/V)). L'extrait brut de *P. parasitica* a été préparé à partir d'un mycélium âgé de deux semaines provenant de la souche 329 de *P. parasitica* (INRA, Sophia-Antipolis). Le mycélium a été lavé à l'eau, broyé en une fine poudre dans un mortier et sous azote liquide, puis ensuite soumis à lyophilisation. Pour sélectionner les micro-organismes eucaryotes, les boîtes de Petri sont supplémentées avec 30µg/mL de chloramphénicol.

Une trentaine d'isolats ont été identifiés comme micro-organismes eucaryotes constituants du biofilm et capables de croitre sur le milieu ainsi préparé, parmi lesquels les isolats notés I1, I2, I3 font l'objet d'une analyse plus détaillée ci-après.

Par isolat on entend au moins une colonie de micro-organismes capables de croitre sur le milieu préparé comme décrit ci-dessus, lesdits micro-organismes étant identiques entre eux.

### 1.3. Identification moléculaire (figure 3)

Pour chacun des isolats, un échantillon de cellules de micro-organismes a été prélevé et soumis à une analyse moléculaire par PCR.

L'échantillon a été préparé en mettant en suspension des cellules, des spores et les mycéliums dans de l'eau bouillante pendant 3 minutes, suivi par un refroidissement rapide avec de la glace et une centrifugation à 10 000xg pendant 3 minutes pour éliminer les débris.

Le surnageant (1µL) a été soumis à une amplification par PCR de l'ADN codant pour l'ARN ribosomique 18S eucaryotique en utilisant l'amorce avant EukA ou SEQ ID N°2 : 5'-CTGGTTGATCCTGCCAG-3' et l'a morce arrière EukB ou SEQ ID N°3 :5'-TGATCCTTCYGCAGGTTC-3' (G. Petroni, F. Dini, F. Verni, G. Rosati, Mol. Phylogenet. Evol. 22, 118-130 (2002)).

Le programme de PCR a inclus une dénaturation initiale à 94°C pendant 120 secondes, suivit par 35 cycles de dénaturation à 94°C pendant 30 secondes, puis une hybridation à 56°C pendant 45 secondes et extension à 72°C pendant 120 secondes.

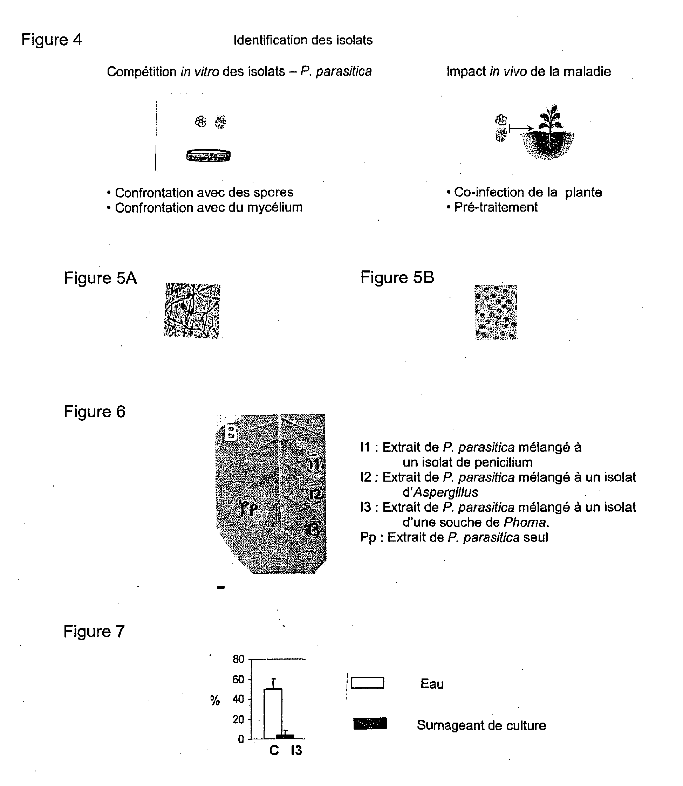

### 1.4. Identification des micro-organismes ayant un impact sur la croissance de P. parasitica et sur la maladie de la plante. (figures 4, 5A, 5B, 6, 7)

Cette identification a été réalisée d'une part, *in vitro* (figures 4, 5A, 5B, 7) en confrontant les micro-organismes représentatifs de chacun des isolats obtenus précédemment et deux souches de *P. parasitica,* et d'autre part aussi *in planta* (figure 4, 6) au moyen de co-infections.

Pour les confrontations *in vitro,* les souches de *P*. *parasitica* et les micro-organismes représentatifs de chaque isolat précédemment obtenu ont été mis en culture sur de l'agar V8. Puis les disques mycéliens obtenus (5mm de diamètre) ont été transférés dans une nouvelle boîte de Petri contentant de l'agar V8 et ont été placés sur la partie droite pour *P*. *parasitica* et sur la partie de gauche pour l'isolat. La zone d'inhibition que l'on voit autour du disque de l'isolat a été utilisée pour mesurer l'activité anti-*P*. *parasitica.*

L'effet des micro-organismes provenant de chacun des isolats I1, I2 et I3 sur la germination de *P*. *parasitica* a aussi été testé, sur lames de microscopie. Une suspension de zoospores (10 µL) de *P*. *parasitica* (4.10⁵ cellules/mL) a été mélangée avec un volume égal de milieu V8 et d'eau préalablement incubée avec chaque isolat testé, puis filtrée. L'eau ainsi conditionnée est préparée en incubant des disques mycéliens dans de l'eau stérile (1 mL) pendant une heure à 25°C. Après une centrifugation à 2000g pendant 2 minutes, le surnageant a été filtré au travers d'un filtre de porosité 0,2 µm pour éliminer les débris et le matériel cellulaire résiduels. La centrifugation peut également être réalisée à une valeur s'étendant dans une fourchette de valeurs allant de 1500g à 6000g et de 1 minute à 10 minutes. De préférence, la centrifugation s'effectue à 2000g pendant 2 minutes.

Le surnageant peut être filtré ou non filtré. Il est généralement filtré après la centrifugation pour se débarrasser des débris cellulaires qui auraient pu se mêler au surnageant au moment de son prélèvement.

La suspension obtenue après incubation des disques mycéliens dans de l'eau stérile pendant 1 heure peut également ne pas subir de centrifugation et être directement filtrée avec le filtre de porosité de 0,2µm.

Le filtre peut présenter une porosité d'une valeur qui peut aussi varier dans une fourchette de valeurs s'étendant de 0,18µm à 0,22µm. de préférence, le filtre présente une porosité de 0,2µm.

Les figures 5A et 5B illustrent une expérience réalisée avec le micro-organisme représentatif de l'isolat 13.

Le pourcentage de germination a été déterminé après une incubation de deux heures des zoospores en présence du surnageant filtré provenant de l'isolat 13 à 25°C, figure 7.

Pour l'analyse et le screening *in planta* (figure 6), la co-infection a été réalisée dans le tissu parenchymateux, une suspension de 100 µL contenant 500 zoospores de *P. parasitica* et 500 spores du micro-organisme représentatif de chaque isolat est infiltrée dans la partie droite des feuilles de tabac âgées de 4 à 6 semaines. Notamment, sur la figure 6 est illustré l'effet du micro-organisme représentatif de chacun des trois isolats I1, 12, I3 sur la feuille. Les aires montrant les symptômes typiques d'invasion par *Phytophthora* ont été mesurées 2 jours après la co-inoculation. Pour évaluer l'impact des micro-organismes représentatifs de chacun des isolats sur la progression de l'infection, ces aires ont été comparées à celles mesurées à la partie gauche de la feuille qui a été inoculée avec 500 zoospores de *P*. *parasitica* seul. Il faut noter qu'aucun micro -organisme présents dans chacun des isolats ne provoque le développement de symptômes sur la plante lorsqu'ils sont inoculés seuls sans zoospores de *P. parasitica* (pendant 7 jours), ni de manifestation de phytotoxicité.

### 1.5. Identification de l'ARNr 18S des micro-organismes de chacun des isolats I1, I2 et I3.

### 1.5.1. Amplification par PCR des ARNr 18S

L'ARNr 18S des micro-organismes correspondant à chacun des isolats 11, I2 et I3 a été amplifié par la technique de PCR. Pour ce faire, il a été utilisé, comme précédemment décrit, une amorce sens de séquence nucléotidique SEQ ID N°2 et une amorce anti-sens de séquence nucléotidique SEQ ID N°3.

### 1.5.2. Identitication des micro-organismes

Les amplifiats correspondant aux ARNr 18S ont été clonés dans le vecteur pGEMT-easy (Promega), séquencés et comparées aux séquences déjà répertoriées dans les banques de données à l'aide du programme blastn (htto:1/blast.ncbi.nlm.nih.gov.gate1.inist.fr/Blast.cgi?PROGRAM=blastn&BLA ST PROGRAMS=megaBlast&PAGE TYPE=BlastSearch&SHOW DEFAUL TS=on&LINK LOC=blasthome)

### 2 - Résultats

Après analyse des séquences nucléotidiques d'ARNr 18S amplifiées; le micro-organisme présent dans l'isolat I1 a été identifié comme un *Penicillium.* Le micro-organisme présent dans l'isolat I2 est un *Aspergillus.* Le micro-organisme présent dans l'isolat I3 est un *Phoma.*

La figure 5B montre l'absence de filaments mycéliens produits par *P*. *parasitica* en présence du surnageant de culture filtré de micro-organismes représentatifs de l'isolat 13. Ainsi, les inventeurs ont constaté que le surnageant de culture filtré issu de l'isolat I3 inhibe la croissance de *P*. *parasitica.*

D'après l'aspect de la feuille de tabac obtenue après co-infection (figure 6), les inventeurs ont constaté également que, à l'endroit où ont été inoculés les spores des isolats I1 et I2 mélangés à *P. parasitica* et *P*. *parasitica* seul, les symptômes de la maladie causée par *P. parasitica* apparaissent. Seul l'endroit où ont été inoculées les spores de l'isolat I3 avec *P. parasitica* ne présente pas ces mêmes symptômes. Par cette expérience, les inventeurs ont confirmé que le micro-organisme présent dans l'isolat I3 présente une activité inhibitrice sur la croissance de *P. parasitica* dans la plante.

Du fait de cette activité inhibitrice potentiellement intéressante pour la lutte biologique contre l'infection des végétaux par *P. parasitica,* le micro-organisme présent dans l'isolat I3 a été identifié. Une étude morphologique au microscope montre qu'il s'agit d'un champignon filamenteux dont les spores sont de couleur brune- marron. La séquence nucléotidique codant pour l'ARNr 18S de ce micro-organisme ou SEQ ID N°1 présente 98% d'identité de séquence avec une séquence nucléotidique codant l'ARNr 18S de la souche *Phoma herbarum.*

Des résultats exposés il découle que cette nouvelle souche de *Phoma* présente dans cet isolat I3 et/ou le surnageant de culture de cette même souche peuvent donc servir de base pour l'élaboration d'une composition phytosanitaire en vue de traiter des végétaux infectés ou susceptibles de l'être par au moins un oomycète phytopathogène.

### Références

J.W. Costerton, P.S. Stewart, E.P. Greenberg, Science 21, 1318-1322 (1999).
T. Danhom, C. Fuqua, Annu Rev. Microbiol. (2007).
A. D. Kent, E. W. Triplett, Annu. Rev. Microbiol. 56, 211-236 (2002).
B. Stecher, W. D. Hardt, Trends Microbiol. 16, 107-14 (2008).
J. Wolinska, K.C. King, Trends Parasitol. 25, 236-244 (2009).
E. Galiana, S. Fourré, G. Engler, Environ. Microbiol. 10, 2164-2171 (2008).
S. Kamoun, Annu. Rev. Phytopathol. 44, 41-60 (2006).
C. Darwin, *John Murray, London,* 67p (1859).
S.J. Gould, Belknap (Harvard University, 473-474p (2002).
E. Galiana; S. Fourré, G. Engler, Environ. Microbiol. 10, 2164-2171 (2008).
G. Petroni, F. Dini, F. Verni, G. Rosati, Mol. Phylogenet. Evol. 22,118-130 (2002).
S. Ischii, T. Shimoyama, Y. Hotta, K. Watanabe, BMC Microbiol. 8, 6 (2008).

## Revendications

1. Souche de *Phoma* déposée à la Collection Nationale de Cultures de micro-organismes de l'Institut Pasteur le 25 février 2010 sous le n°CNCM I-4278.

2. Souche selon la revendication 1, **caractérisée en ce qu'**elle inhibe au moins partiellement la croissance des oomycètes phytopathogènes.

3. Souche selon la revendication 1, **caractérisée en ce qu'**elle inhibe la croissance des oomycètes du genre *Phytophthora.*

4. Souche selon la revendication 3, **caractérisée en ce qu'**elle inhibe la croissance de *Phytophthora parasitica.*

5. Surnageant de culture de la souche selon la revendication 1, **caractérisé en ce que** ledit surnageant inhibe au moins partiellement la croissance des oomycètes phytopathogènes.

6. Surnageant selon la revendication 5, **caractérisé en ce qu'**il comporte au moins un métabolite de taille inférieure ou égale à une valeur comprise entre 0,18µm et 0,22µm, de préférence inférieure ou égale à 0,2µm.

7. Surnageant selon l'une des revendications 5 à 6, **caractérisé en ce que** le surnageant de culture inhibe la croissance de *Phytophthora parasitica.*

8. Procédé d'obtention du surnageant selon l'une des revendications 5 à 7, ledit procédé comprenant les étapes suivantes
- mise en culture de la souche selon la revendication 1,
- mise en suspension de la culture obtenue dans l'eau, **caractérisé en ce qu'**il comporte l'étape suivante
- filtration de la suspension au travers d'un tamis de 0,18µm à 0,22µm, de préférence 0,2µm,
- récupérer la solution filtrée.

9. Procédé selon la revendication 8, **caractérisé en ce qu'**il comporte les étapes suivantes
- préalablement à la filtration de la suspension, centrifuger la suspension entre 1500g et 6000g pendant 1 à 10 minutes, de préférence à 2000g pendant 2 minutes.

10. Procédé d'obtention du surnageant selon l'une des revendications 5 à 7, comprenant les étapes suivantes
- mise en culture de la souche selon la revendication 1,
- mise en suspension dans l'eau de la culture obtenue, **caractérisé en ce qu'**il comporte l'étape suivante
- centrifuger la suspension entre 1500g et 6000g pendant 1 à 10 minutes, de préférence à 2000g pendant 2 minutes, puis
- récupérer le surnageant.

11. Composition phytosanitaire, **caractérisée en ce qu'**elle comprend la souche de *Phoma* selon la revendication 1.

12. Composition phytosanitaire, **caractérisée en ce qu'**elle comprend un surnageant de culture selon l'une des revendications 5 à 7.

13. Composition phytosanitaire, **caractérisée en ce qu'**elle comprend la composition selon la revendication 11 et la composition selon la revendication 12.

14. Utilisation de la souche de *Phoma* selon la revendication 1 pour la fabrication d'une composition phytosanitaire destinée à traiter des végétaux contre l'infection par des oomycètes phytopathogènes.

15. Utilisation d'un surnageant de culture selon l'une des revendications 5 à 7 pour la fabrication d'une composition phytosanitaire destinée à traiter des végétaux contre l'infection par des oomycètes phytopathogènes.

16. Utilisation de la souche de *Phoma* selon la revendication 1 et du surnageant de culture selon l'une des revendications 5 à 7 pour la fabrication d'une composition phytosanitaire destinée à traiter des végétaux contre l'infection par des oomycètes phytopathogènes.

## Patentansprüche

1. Stamm *Phoma,* der am 25. Februar 2010 unter der Nummer CNCM I-4278 in der Collection Nationale de Cultures de Micro-organismes im Institut Pasteur hinterlegt wurde.

2. Stamm nach Anspruch 1, **dadurch gekennzeichnet, dass** er das Wachstum der pathogenen Eipilze zumindest teilweise unterbindet.

3. Stamm nach Anspruch 1, **dadurch gekennzeichnet, dass** er das Wachstum der Eipilze der Art *Phytophthora* unterbindet.

4. Stamm nach Anspruch 3, **dadurch gekennzeichnet, dass** er das Wachstum von *Phytophthora parasitica* unterbindet.

5. Kulturüberstand des Stamms nach Anspruch 1, **dadurch gekennzeichnet, dass** der besagte Überstand das Wachstum der pathogenen Eipilze zumindest teilweise unterbindet.

6. Überstand nach Anspruch 5, **dadurch gekennzeichnet, dass** er zumindest einen Metaboliten enthält, dessen Größe kleiner oder gleich einem Wert zwischen 0,18 µm und 0,22 µm, vorzugsweise kleiner oder gleich 0,2 µm ist.

7. Überstand nach einem der Ansprüche 5 bis 6, **dadurch gekennzeichnet, dass** der Kulturüberstand das Wachstum von *Phytophthora parasitica* unterbindet.

8. Verfahren zum Erhalt des Überstands nach einem der Ansprüche 5 bis 7, wobei das besagte Verfahren die folgenden Schritte umfasst
- das Ansetzen des Stammes nach Anspruch 1,
- das Suspendieren der erhaltenen Kultur im Wasser, **dadurch gekennzeichnet, dass** es den folgenden Schritt umfasst
- das Filtern der Suspension durch ein Sieb von 0,18 µm bis 0,22 µm, vorzugsweise von 0,2 µm,
- das Auffangen der gefilterten Lösung.

9. Verfahren nach Anspruch 8, **dadurch gekennzeichnet, dass** es die folgenden Schritte umfasst
- vor dem Filtern der Suspension das Zentrifugieren der Suspension 1 bis 10 Minuten lang zwischen 1500g und 6000g, vorzugsweise 2 Minuten lang bei 2000g.

10. Verfahren zum Erhalt des Überstands nach einem der Ansprüche 5 bis 7, das die folgenden Schritte umfasst
- das Ansetzen des Stammes nach Anspruch 1,
- das Suspendieren der erhaltenen Kultur im Wasser, **dadurch gekennzeichnet, dass** es den folgenden Schritt umfasst
- das Zentrifugieren der Suspension 1 bis 10 Minuten lang zwischen 1500g und 6000g, vorzugsweise 2 Minuten lang bei 2000g, danach
- das Auffangen des Überstands.

11. Pflanzenschutz-Zusammensetzung, **dadurch gekennzeichnet, dass** sie den Stamm *Phoma* nach Anspruch 1 enthält.

12. Pflanzenschutz-Zusammensetzung, **dadurch gekennzeichnet, dass** sie einen Kulturüberstand nach einem der Ansprüche 5 bis 7 enthält.

13. Pflanzenschutz-Zusammensetzung, **dadurch gekennzeichnet, dass** sie die Zusammensetzung nach Anspruch 11 und die Zusammensetzung nach Anspruch 12 enthält.

14. Verwendung des Stamms *Phoma* nach Anspruch 1 zur Herstellung einer Pflanzenschutz-Zusammensetzung, die dazu bestimmt ist, Pflanzen gegen die Infektion mit pathogenen Eipilzen zu behandeln.

15. Verwendung eines Kulturüberstands nach einem der Ansprüche 5 bis 7 zur Herstellung einer Pflanzenschutz-Zusammensetzung, die dazu bestimmt ist, Pflanzen gegen die Infektion mit pathogenen Eipilzen zu behandeln.

16. Verwendung des Stamms *Phoma* nach Anspruch 1 und des Kulturüberstands nach einem der Ansprüche 5 bis 7 zur Herstellung einer Pflanzenschutz-Zusammensetzung, die dazu bestimmt ist, Pflanzen gegen die Infektion mit pathogenen Eipilzen zu behandeln.

## Claims

1. Strain of *Phoma* registered at the Collection Nationale de Cultures de micro-organismes of the Pasteur Institute on February 25 2010 under the CNCM number I-4278.

2. Strain according to claim 1, **characterized in that** it inhibits, at least partially, the growth of the phytopathogenic oomycetes.

3. Strain according to claim 1, **characterized in that** it inhibits the growth of the oomycetes of the genus *Phytophthora.*

4. Strain according to claim 3, **characterized in that** it inhibits the growth of *Phytophthora parasitica.*

5. Culture supernatant of the strain according to claim 1, **characterized in that** said supernatant inhibits, at least partially, the growth of the phytopathogenic oomycetes.

6. Supernatant according to claim 5, **characterized in that** it comprises at least one metabolite having a size lower than or equal to a value between 0.18 µm and 0.22 µm, preferably lower than or equal to 0.2 µm.

7. Supernatant according to any one of claims 5 to 6, **characterized in that** the culture supernatant inhibits the growth of *Phytophthora parasitica.*

8. Method for obtaining the supernatant according to any one of claims 5 to 7, said method including the following steps of
- cultivating the strain according to claim 1,
- suspending the culture obtained in water, **characterized in that** it comprises the following step of
- filtering the suspension through a sieve from 0.18 µm to 0.22 µm, preferably of 0.2 µm,
- collecting the filtered solution.

9. Method according to claim 8, **characterized in that** it comprises the following steps of
- before the filtering of the suspension, centrifuging the suspension between 1500 g and 6000 g for 1 to 10 minutes, preferably at 2000 g for 2 minutes.

10. Method for obtaining the supernatant according to any one of claims 5 to 7, comprising the following steps of
- cultivating the strain according to claim 1,
- suspending the culture obtained in water, **characterized in that** it comprises the following step of
- centrifuging the suspension between 1500 g and 6000 g for 1 to 10 minutes, preferably at 2000 g for 2 minutes, then
- collecting the supernatant.

11. Plant-care composition, **characterized in that** it includes the strain of *Phoma* according to claim 1.

12. Plant-care composition, **characterized in that** it includes a culture supernatant according to any one of claims 5 to 7.

13. Plant-care composition, **characterized in that** it includes the composition according to claim 11 and the composition according to claim 12.

14. Use of the strain of *Phoma* according to claim 1 for manufacturing a plant-care composition intended to treat plants against infection by phytopathogenic oomycetes.

15. Use of a culture supernatant according to any one of claims 5 to 7 for manufacturing a plant-care composition intended to treat plants against infection by phytopathogenic oomycetes.

16. Use of the strain of *Phoma* according to claim 1 and of the culture supernatant according to any one of claims 5 to 7 for manufacturing a plant-care composition intended to treat plants against infection by phytopathogenic oomycetes.


### SEQUENCE LISTING

<110> INSTITUT NATIONAL DE LA RECHERCHE AGRONOMIQUE
<120> Traitement des végétaux contre l'infection par dés oomycètes
<130> 24346WO
<150> FR n°10 51767
   <151> 2010-03-11
<160> 3
<170> PatentIn version 3.5
<210> 1
   <211> 1793
   <212> DNA
   <213> Phoma
<400> 1
   ctggttgatc ctgccagtag tcatatgctt gtctcaaaga ttaagccatg catgtctaag      60
   tataagcaat tataccgtga aactgcgaac ggctcattaa atcagttatc gtttatttga     120
   tagtacctta ctacttggat aaccgtggta attctagagc taatacatgc taaaaacctc     180
   gacttcggga ggggtgtatt tattagataa aaaaccaatg cccttcgggg ctctctggtg     240
   attcataata acttctcaga tcgcatggcc ttgcgccggc gacggttcat tcaaatttct     300
   gccctatcaa ctttcgatgg taaggtattg gcttaccatg gtttcaacgg gtaacgggga     360
   attagggttc gattccggag agggagcctg agaaacggct accacatcca aggaaggcag     420
   caggcgcgca aattacccaa tcccaatacg gggaggtagt gacaataaat actgatacag     480
   ggctctttag ggtcttgtaa ttggaatgag tacaatttaa acctcttaac gaggaacaat     540
   tggagggcaa gtctggtgcc agcagccgcg gtaattccag ctccaatagc gtatattaaa     600
   gttgttgcag ttaaaaagct cgtagttgaa actttggcct ggctggcggg tccgcctcac     660
   cgcgtgcatt cgcccggccg ggccttttct tctggagaac cgcatgccct tcactgggtg     720
   tgttggggac caggactttt actttgaata aatcagagtg ttcaaagcag gcatttgctc     780
   gaatacgtta gcatggaata atagaatagg acgtgcggtc ttattttgtt ggtttctaag     840
   accgccgtaa tgattaatag ggacagtcgg gggcatcagt attcaattgt cagaggtgaa     900
   attcttggat ttattgaaga ctaactactg cgaaagcatt tgccaaggat gttttcatta     960
   atcagtgaac gaaagttagg ggatcgaaga cgatcagata ccgtcgtagt cttaaccata    1020
   aactatgccg actagggatc gggcggtgtt actattttga ctcgctcggc accttacgag    1080
   aaatcaaagt gtttgggttc tggggggagt atggtcgcaa ggctgaaact taaagaaatt    1140
   gacggaaggg caccaccagg cgtggagcct gcggcttaat ttgactcaac acggggaaac    1200
   tcaccaggtc cagatgaaat aaggattgac agattgagag ctctttcttg atttttcagg    1260
   tggtggtgca tggccgttct tagttggtgg agtgatttgt ctgcttaatt gcgataacga    1320
   acgagacctt aacctgctaa atagccaggc tagctttggc tggtcgccgg cttcttagag    1380
   ggactatcgg ctcaagccga tggaagtttg aggcaataac aggtctgtga tgcccttaga    1440
   tgttctgggc cgcacgcgcg ctacactgac agagccaacg agtttttttc cttggccgaa    1500
   aggcctgggt aatcttgtta aactctgtcg tgctggggat agagcattgc aattattgct    1560
   cttcaacgag gaatgcctag taagcgcgtg tcatcagcac gcgttgatta cgtccctgcc    1620
   ctttgtacac accgcccgtc gctactaccg attgaatggc tcagtgaggc cttcggactg    1680
   gctcgaggag gttggcaacg accaccctga gccggaaagt tcgtcaaact cggtcattta    1740
   gaggaagtaa aagtcgtaac aaggtttccg taggtgaacc tgcagaagga tca           1793
<210> 2
   <211> 17
   <212> DNA
   <213> Eucaryote
<400> 2
   ctggttgatc ctgccag 17
<210> 3
   <211> 18
   <212> DNA
   <213> Eucaryote
<400> 3
   tgatccttcy gcaggttc 18